# EUROPEAN PATENT SPECIFICATION

(11) **EP 2 146 755 B1**
(45) Date of publication and mention of the grant of the patent: **30.03.2016**
(21) Application number: 07728321.6
(22) Date of filing: 19.04.2007
(51) Int. Cl.: A61L 27/20, A61L 27/54, A61L 27/58, A61L 29/14, A61L 29/16, A61L 31/04, A61L 31/14, A61L 31/16

(54) **DEVICE MADE AT LEAST PARTIALLY OF N-ACETYLCHITOSAN WITH CONTROLLED BIODISSOLUTION**
ZUMINDEST TEILWEISE AUS N-ACETYLCHITOSAN BESTEHENDE VORRICHTUNG MIT KONTROLLIERTER BIOLOGISCHER AUFLÖSUNG
DISPOSITIF FABRIQUÉ AU MOINS PARTIELLEMENT À PARTIR DE N-ACÉTYLCHITOSANE AVEC UNE BIODISSOLUTION CONTRÔLÉE

(43) Date of publication of application: 27.01.2010
(73) Proprietor: Medovent Gmbh, 55131 Mainz (DE)
(72) Inventor: FREIER, Thomas, 55130 Mainz (DE); MONTENEGRO, Rivelino, 55129 Mainz (DE)
(74) Representative: Huebner, Stefan Rolf
(86) International application number: PCT/EP2007/053862
(87) International publication number: WO 2008/128567

(56) References cited:
- EP-A- 0 296 078
- WO-A-02/03962
- WO-A-2004/026358
- WO-A-2007/042281
- US-A1- 2005 176 678
- AIBA ET AL: "Studies on chitosan: 3. Evidence for the presence of random and block copolymer structures in partially N-acetylated chitosans", INTERNATIONAL JOURNAL OF BIOLOGICAL MACROMOLECULES, ELSEVIER BV, NL, vol. 13, no. 1, 1 February 1991 (1991-02-01), pages 40-44, XP023450022, ISSN: 0141-8130, DOI: 10.1016/0141-8130(91)90008-I [retrieved on 1991-02-01]
- AIBA S-I: "STUDIES ON CHITOSAN 4. LYSOZYMIC HYDROLYSIS OF PARTIALLY N-ACETYLATED CHITOSANS", INTERNATIONAL JOURNAL OF BIOLOGICAL MACROMOLECULES, vol. 14, no. 4, 1992, pages 225-228, ISSN: 0141-8130

## Description

### FIELD OF THE INVENTION

The present invention is related to medical devices based on N-acetylchitosan that can be dissolved in a highly controllable fashion when implanted or inserted in a patient's body.

### BACKGROUND OF THE INVENTION

Chitin and chitosan represent a family of biopolymers, made up of N-acetyl-D-glucosamine and D-glucosamine subunits. Chitin can be found widely in the exoskeletons of arthropods, shells of crustaceans, and the cuticles of insects. Chitosan, although occurring in some fungi, is produced industrially by alkaline hydrolysis of chitin. Their different solubilities in dilute acids are commonly used to distinguish between both polysaccharides. Chitosan, the soluble form, can have a degree of acetylation between 0% and about 60%, the upper limit depending on parameters such as processing conditions, molecular weight, and solvent characteristics.

Both chitin and chitosan are promising polymers for a variety of applications, including water treatment (metal removal, flocculant/coagulant, filtration), pulp and paper (surface treatment, photographic paper, copy paper), cosmetics (make-up powder, nail polish, moisturizers, fixtures, bath lotion, face, hand and body creams, toothpaste, foam enhancing), biotechnology (enzyme immobilization, protein separation, chromatography, cell recovery, cell immobilization, glucose electrode), agriculture (seed coating, leaf coating, hydroponic/fertilizer, controlled agrochemical release), food (removal of dyes, solids and acids, preservatives, color stabilization, animal feed additive), and membranes (reverse osmosis, permeability control, solvent separation). Of particular interest are biomedical applications of chitin and chitosan because of their biocompatibility, biodegradability and structural similarity to the glycosaminoglycans. Applications and potential applications include wound dressings, tissue engineering applications, artificial kidney membranes, drug delivery systems, absorbable sutures, hemostats, antimicrobial applications, as well as applications in dentistry, orthopedics, ophthalmology, and plastic surgery. For comprehensive reviews of potential applications of chitin and chitosan see, for example, Applications of chitin and chitosan, 1997; Shigemasa and Minami, Biotech Genetic Eng Rev 1996;13:383-420; Ravi Kumar, React Funct Polym 2000;46:1-27; Singh and Ray, J Macromol Sci 2000;C40:69-83.

The disintegration of a medical device made of chitin or chitosan when implanted or inserted in a patient's body can be due to a biodegradation (depolymerization) and/or biodissolution process. It is well known that, for example, in human serum, chitin and chitosan are mainly depolymerized enzymatically by lysozyme, and not by other enzymes or other depolymerization mechanisms. The enzyme biodegrades the polysaccharide by hydrolyzing the glycosidic bonds present in the chemical structure. Lysozyme contains a hexameric binding site, and hexasaccharide sequences containing 3-4 or more acetylated units contribute mainly to the degradation rate. While the concentration of lysozyme is high in a number of human body fluids, such as tears, gastric juice, sperm, serum, amniotic fluid, and saliva, it is negligable if undetectable in cerebrospinal fluid, urine, bile, and feces.

In contrast to the biodegradation mechanism, the biodissolution of chitosan is mainly controlled by its degree of acetylation (DA) and molecular weight, as well as the availability of liquid and the pH at the application site. It is well known, for example, that chitosan becomes readily soluble already in neutral water when the DA is close to 50%. The enzymatic hydrolysis, which can be expected to increase with increasing DA due to the increasing availability of acetylated units, is therefore overshadowed by the enhanced solubility of chitosan with intermediate DAs, which results in an accelerated mass loss.

Both the biodegradation and biodissolution processes of chitin and chitosan depend, as outlined above, on a number of parameters that may be difficult to control under physiological conditions. However, in most cases, it is highly desirable to predict or control the disintegration process of a biodegradable or biodissolvable medical device. This particularly applies to tubular implants, such as stents or catheters, which disintegration should not be accompanied by a significant swelling of the tube wall, causing tissue compression and irritation at the site of implantation, nor blockage of the tube lumen, leading to loss of functionality of the device. Additionally, any obstruction of an opening inside the body due to swelling of the degrading tube or due to fragments or particles that are cleaved off should be avoided. It would be highly desirable to allow for a surface dissolution instead of bulk degradation mechanism to prevent the aforementioned complications. Surface dissolution (erosion) of a tubular device would lead to a continuous decrease in the wall thickness thereby avoiding tube swelling and lumen obstruction, and it will not cause voluminous fragments to be formed in the course of disintegration.

Few approaches have been described to fabricate medical devices made of chitosan than can be degraded and/or dissolved in a controllable manner. For example, US 5,531,735 to Thompson describes a combination of a matrix polymer, such as chitosan, which is essentially insoluble in body fluids, with a disintegration agent, such as lysozyme, which is isolated from the matrix polymer by encapsulation in an ionically crosslinked second polymer or by presence in an interpolyelectrolyte complex. The degradation of a chitosan tube exemplified in '735 is triggered by displacing crosslinking ions present in the ionically crosslinked second polymer (alginate) thereby releasing lysozyme capable of disintegrating the chitosan tube. However, in this assembling, a secondary disintegration process has to be triggered and controlled in order to initiate the disintegration of the primary target device which may be difficult under physiological conditions. Moreover, the implantation of an enzyme in a patient may cause foreign-body reactions, and the enzymatic activity may be significantly affected and reduced at the implantation site.

In the co-pending unpublished International Patent Application PCT/EP2006/009830 to Freier, there are described ureteral stents based on N-acetylchitosan that have been hydrolyzed up to three times to achieve a pH- dependent dissolution mechanism which allows these stents to be removed from the patient's body in a highly controllable fashion, by adjusting the pH of the patient's urine, which can be done by treatment with basic or acidic compounds added to the diet. Stents that have been hydrolyzed three times showed complete dissolution in vitro after 2 days of storage in human urine, and stents that have been hydrolyzed one time only dissolved within three to twelve days, depending on the application of a coating layer to the stent surface. A gel-like dissolution associated with tube swelling has been reported in these experiments.

In "Chitin-based tubes for tissue engineering in the nervous system", Biomaterials 2005; 26; pages 4624-4632, Freier et al. describe biodegradable nerve guides made of N-acetylchitosan with degrees of acetylation of 1%, 3% and 18%.

The international patent application WO 2004/026358 A1 discloses a reinforcing plate for medical uses, the reinforcing plate comprising chitosan fibres with a degree of acetylation of less than 60%. It is mentioned that chitosan fibres with a degree of acetylation below 5% are only soluble at a pH between 1 and 6.

The US Patent application US 2005/0176678 A1 suggests that partially N-carboxymethylated and N-acetylated chitosan with a degree of acylation between 30% and 70% can be useful in the manufacture of stents, in particular as a hemo-compatible coating. Moreover, from the European patent application EP 0 296 078 A1 a biomaterial is known that contains chitosan with a degree of acetylation between 10 and 40%.

Finally, the international patent application WO 2002/03962 A2 discloses a drug delivery system comprising a chitosan-xanthan hydrogel. Upon swelling of the hydrogel, a drug incorporated into the hydrogel becomes at least partly solubilised and thereby released from the hydrogel.

The present invention describes devices, particularly medical devices, such as stents and catheters, that are based on N-acetylchitosan that have moderate DAs. The biodissolution of these devices takes place by surface-erosion, without the formation of obstructive fragments. The N-acetylchitosan devices of the present invention are designed in a way that they become biodissolvable at moderate acidic pH of the environment they are in contact with so that the process of biodissolution can be triggered simply by adjusting the pH of a fluid or tissue leading to disintegration of the device in a highly controllable fashion.

### SUMMARY OF THE INVENTION

In the description of the present invention, the term "chitin" is used for a naturally derived polymer made up of N-acetyl-D-glucosamine and D-glucosamine subunits that is non-soluble in dilute acids. The term "chitosan" is used for a polymer made up of either N-acetyl-D-glucosamine subunits or N-acteyl-D-glucosamine and D-glucosamine subunits that is either naturally derived or synthetically prepared by hydrolysis of chitin and that is soluble in dilute acids. The term "N-acetylchitosan" represents a polymer that is synthetically prepared by N-acetylation of chitosan or that is synthetically prepared by hydrolysis of an N-acetylchitosan prepared by N-acetylation of chitosan. The term "N-acetylchitosan hydrogel" is used for an N-acetylchitosan network that is swollen in an aqueous environment. The term "biodissolution" of a material or device describes the process of mass loss without molecular weight decrease due to solubility in an aqueous environment while "biodegradation" is the process of molecular weight decrease due to depolymerisation of a material or device.

It is an object of the present invention to provide an improved stent or catheter, and an improved drug delivery device.

In accordance with the present invention, there is provided a stent, a catheter or a drug delivery device according to claim 1.

Moreover, according to the present invention there are provided uses of N-acetylchitosan for the manufacture of a stent, a catheter, or a drug delivery device according to claim 2.

It is an achievable advantage of the present invention that the device comprising N-acetylchitosan can be biodissolved by a controllable process.

It is further an achievable advantage of the present invention that the device comprising N-acetylchitosan can be biodissolved by a surface-erosion process.

It is further an achievable advantage of the present invention that the device comprising N-acetylchitosan can be biodissolved completely.

It is a further achievable advantage of the present invention that the device can be biodissolved within a relatively short period of time. The inventor observed complete dissolution within less than two days or even within less than 24 h. This can be advantageous in various medical applications, e.g. when the invention is applied to a ureteral stent.

It is further an achievable advantage of the present invention that the device comprising N-acetylchitosan can be biodissolved in contact with a body fluid.

It is further an achievable advantage of the present invention that the device comprising N-acetylchitosan can be biodissolved by adjusting the pH of a body fluid.

It is further an achievable advantage of the present invention that the device comprising N-acetylchitosan is in the shape of a tube.

The degree of acetylation can be measured by the method disclosed in Freier et al., "Chitin-based tubes for tissue engineering in the nervous system" Biomaterials 2005; 26; page 4625; section 2.2 with reference to Vachoud et al., "Formation and characterisation of a physical chitin gel" Carbohydr. Res. 1997; 302; pages 169-177 and Lavertu et al., "A validated 1 H NMR method for the determination of the degree of deacetylation of chitosan"; J. Pharm. Biomed. Anal. 2003; 32; pages 1149-1158.

The aqueous medium preferably is a physiological medium. It may be of natural origin or it may be artificial, preferably imitating a natural physiological medium, e.g. artificial urine. A preferred physiological medium has the composition of a body fluid, e.g. urine, blood, gastrointestinal fluid, pulmonary fluid, or bile. A physiological medium with the composition of urine in the context of the present invention is an aqueous solution that has a composition as described by McLean et al., "An in vitro ultrastructural study of infectious kidney stone genesis" Infect. Immun. 1985; 49; p. 805 (without usage of tryptic soy broth) with reference to Griffith et al., "Urease - the primary cause of infection-induced urinary stones" Invest. Urol. 1976; 13; p. 346-350, or one of the compositions of urine defined in ASTM F1828-97 (2006), p. 6. with reference to Burns et al., "Proposal for a standard reference artificial urine in in-vitro urolithiasis experiments" Invest. Urol. 1980; 18; pages 167-169 and British Standard 1695, "Urological catheters, Part 2: Specification for sterile, single-use urethral catheters of the Tiemann, whistle-tip, 3-way, and haematuria types" Section D.2.4; September 1990.

Another preferred aqueous medium is a diluted acid, e.g. diluted acetic acid, e.g. at a concentration between 0.25% and 2%, or diluted hydrochloric acid, e.g. at a concentration of about 0.25%.

The device may be made completely of N-acetylchitosan with the properties according to the invention, or only partially. The N-acetylchitosan is preferably dissolved by a surface-erosion mechanism.

In one embodiment of the invention, the device is a medical device, preferably one that can be implanted or inserted into a patient's body. However there are also numerous possible application of the invention outside medicine, for example in the manufacture of biodissolvable paper, cosmetics, and coatings, e.g. seed or leaf coatings for agriculture, as well as controlled release systems, e.g. for the controlled release of agrochemicals such as fertilizers. In biotechnology, the invention provides new options in enzyme immobilization, protein separation, chromatography, cell recovery and cell immobilization to name only a few promising applications. The drug delivery device is preferably implanted into the patient, e.g. into the urinary passage. Alternatively, however, it may e.g. be administered orally or injected into the patient, e.g. subcutaneously.

The pH of the aqueous medium is preferably adjusted to equal or less than 6.0 to trigger or control the biodissolution, more preferably equal or less than 5.5. The pH of the aqueous medium is preferably adjusted to equal or more than 1.0 to trigger or control the biodissolution, more preferably equal or more than 2.5, more preferably equal or more than 4.0, more preferably equal or more than 5.0. It may be adjusted periodically between a pH value that is above and a value that is below 6.0, more preferably 5.5. By means of such a periodical adjustment, it is possible to achieve a step-wise disintegration of the device. This may be of particular advantage if the device is used to deliver a therapeutic agent that is released as a result of N-acetylchitosan dissolution.

In a preferred embodiment, the N-acetylchitosan is dissolvable in the aqueous medium - preferably within less than 24 hours, more preferably within less than 12 hours, even more preferably within equal or less than 6 hours - if the pH has any value between 1.0 and 5.5, more preferably if the pH has any value between 2.5 and 5.5, even more preferably if the pH has any value between 4.0 and 5.5, even more preferably if the pH has any value between 5.0 and 5.5. In a preferred embodiment, the N-acetylchitosan is dissolvable in the aqueous medium - preferably within less than 24 hours, more preferably within less than 12 hours, even more preferably within equal or less than 6 hours - if the pH has any value between 1.0 and 6.0, more preferably if the pH has any value between 2.5 and 6.0, even more preferably if the pH has any value between 4.0 and 6.0, even more preferably if the pH has any value between 5.0 and 6.0.

In a preferred embodiment, the N-acetylchitosan is substantially not dissolvable in the aqueous medium if the pH has any value below 1.0, more preferably below 2.5, even more preferably below 4.0, even more preferably below 5.0. In a preferred embodiment, the N-acetylchitosan is substantially not dissolvable in the aqueous medium if the pH has any value above 6.0, more preferably above 5.5. The N-acetylchitosan part of the preferred device results in an effective diffusion coefficient of vitamin B12 of equal or less than 1x10-7 cm2/s, as measured as described in Freier et al., et al., "Chitin-based tubes for tissue engineering in the nervous system" Biomaterials 2005; 26; page 4626, section 2.7.

In a preferred embodiment of the present invention the medical device essentially is sheet- or tube-like, e.g. a stent or catheter, and particularly preferably a ureteral, gastrointestinal, biliary, cardiovascular, or pulmonary stent. Preferred ureteral stents have at least one end in the form of a pigtail, a J-shape or other curved shapes.

### BRIEF DESCRIPTION OF THE DRAWINGS

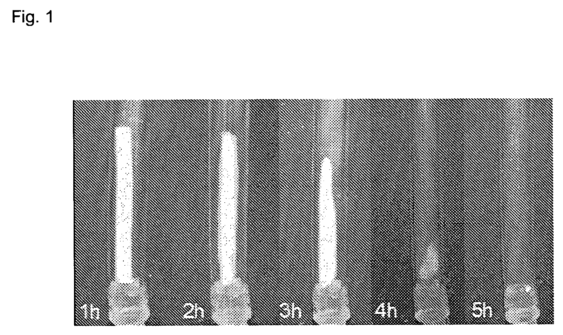

Figure 1 illustrates the controlled dissolution of a tubular device made of N-acetylchitosan (with contrast agent) in artificial urine after changing the pH from 6.5 to 5.0 (sample 116 from table 1).

### DETAILED DESCRIPTION OF THE INVENTION

The invention relates to medical devices based on N-acetylchitosan that can be biodissolved in a highly controllable manner. Medical products which may consist in total or in part of N-acetylchitosan may include sutures, suture fasteners, slings, coils, rivets, tacks, staples, clips, hooks, buttons, snaps, orthopedic pins/clamps/screws/dowels/plates, bone substitutes, spinal cages/plates/rods/screws/discs, finger joints, intramedullary nails, hip prosthesis, meniscus repair devices, knee replacement devices, cartilage repair devices, ligament and tendon grafts, tendon repair devices, surgical mesh, surgical repair patches, hernia patches, pericardial patches, cardiovascular patches, adhesion barriers, abdominal wall prosthesis, catheters, shunts, stents (vascular, urological, gastrointestinal, pulmonary, biliary), vascular grafts and substitutes, coronary artery bypass grafts, guided tissue repair/regeneration devices, scaffolds for tissue engineering, nerve guides, septal defect repair devices, heart valves, vein valves, artificial fallopian tubes, drainage tubes and implants, intrauterine devices, intraocular implants, keratoprosthesis, dental implants, orbital floor substitutes, skin substitutes, dural substitutes, intestinal substitues, fascial substitutes, wound dressings, burn dressings, medicated dressings, gauze/fabric/sheet/felt/sponge for hemostasis, gauze bandages, bandages for skin surfaces, adhesive bandages, bulking and filling agents, drug delivery matrices, injectable gels and systems, and others. The biodissolvable medical devices according to the present invention are particularly applicable for use in urogenital, cardiovascular, gastrointestinal, neurological, lymphatic, otorhinolaryngological, ophthalmological and dental applications. Additionally, they are particularly applicable for tissue engineering. The present invention is particularly applicable to tubular devices, such as stents, which come in contact with body fluids such as urine, blood, gastrointestinal fluids, pulmonary fluids, and bile.

In accordance with the present invention, medical devices based on N-acetylchitosan are made by starting from chitosan that is transformed into N-acetylchitosan gels. The selective N-acetylation reaction of chitosan forming N-acetylchitosan gels is well-known in the art and usually includes the treatment of chitosan, which is dissolved in diluted acidic solution and mixed with a cosolvent, with acetic anhydride. After mixing of the components, gel formation occurs within a few seconds to hours, depending on the reaction conditions and used reactants.

Suitable solvents for chitosan include dilute inorganic and organic acids, such as formic, acetic, propionic, lactic, and citric acid; most preferable is aqueous acetic acid. Suitable cosolvents to be added to the chitosan solution include water as well as organic liquids, such as methanol, ethanol, propanol, butanol, trifluoroethanol, ethylene glycol, diethylene glycol, polyethylene glycol, glycerol, formamide, N,N-dimethyl formamide, N-methylpyrrolidone, dimethyl sulfoxide, dioxane, and tetrahydrofurane.

N-acetylchitosan gels may be made by extrusion or by other processes which are known in the art to fabricate medical devices. Injection molding is the most preferable method among these other processes. Preferably, extrusion involves dissolution of 2-10% chitosan in 0.5-15% aqueous acetic acid, addition of a 1-2.5fold volume of ethanol, and extrusion of the resulting homogeneous mixture into an acetylation bath containing 10-90% acetic anhydride in ethanol. More preferably, chitosan is dissolved in a concentration of 3-5% in 2-5% aqueous acetic acid, mixed with a 1-2fold volume of ethanol, and extruded into an acetylation bath containing 25-50% of acetic anhydride in ethanol. For injection-molding, N-acetylchitosan gels are preferably made by treatment of a solution of 2-5% chitosan in 0.5-10% aqueous acetic acid, the solution being diluted with a 0.5-2fold volume of ethanol, with a 1-3fold excess of acetic anhydride. More preferably, a solution of 3-4% chitosan in 2-5% aqueous acetic acid is mixed with a 1-2fold volume of ethanol, and a 1.5-2.5fold excess of acetic anhydride is added.

In both cases, for extrusion and injection-molding, the chitosan used as starting material has preferably a degree of acetylation of less than 25% and a viscosity between approximately 50-2000 mPas (analyzed as 1% solution in 1% acetic acid on a Brookfield viscometer at 25°C). More preferably, the chitosan has a degree of acetylation of less than 15% and a viscosity between approximately 100-1000 mPas.

N-acetylchitosan gels which are suitable for the fabrication of medical devices may have the shape of a rod, fiber, tube, film, sphere or other geometric structures which may be hollow or not. The gel may already have a shape similar to that of the desired final product. Fibers, tubes, films, and other articles, which may be hollow or not, may be made by extrusion as described above, through a die of pre-selected size and shape. In an injection-molding process, the acetylation reaction mixture may simply be injected into a mold of pre-selected size and shape, and will be left for gelation without further application of any forces, in order to allow for homogeneous gel formation. For example, movement of the mold or application of forces to the mold during gel formation may result in inhomogeneous gel morphologies which is disadvantageous with respect to the formation of medical devices according to the present invention. N-acetylchitosan gel rods and fibers may be fabricated by injecting the acetylation reaction mixture into a cylindrical mold for gel formation. Similarly, N-acetylchitosan gel tubes may be fabricated by injecting the acetylation reaction mixture into a cylindrical mold which contains a centrally fixed core for gel formation. Cylindrical molds may contain more than one core to fabricate gel tubes with multiple channels. Corrugated rods and tubes may be fabricated by using a corrugated mold for injection and gel formation. Similarly, other three-dimensional structures may be fabricated by injecting the acetylation reaction mixture into appropriate molds for gelation. N-acetylchitosan gel films can simply be made by pouring the acetylation reaction mixture into a Petri dish or similar container for gel formation, or by injection into a suitable mold. Another technique is to cut a gel tube longitudinally to provide a film.

Medical devices based on N-acetylchitosan having improved mechanical strength and shape-memory stability may be fabricated by drying N-acetylchitosan gel structures such as those described above under fixation of the desired shape. The collaps of the honeycomb-like morphology of the hydrogel during the dehydration/desolvation process leads to the irreversible preservation of the fixed shape together with improved mechanical stability due to a denser packing of the polymer bulk. The such formed shaped article may be conformable to the shape of a medical device or part of a medical device, including the shape of an anchor, hook, coil, mesh, textile, foam, scaffold, stent, catheter, tube, sphere, particle, plug, plate, screw, pin, tack, clip, ring, drug-release depot, cell-encapsulation device.

N-acetylchitosan gels may be modified prior to the drying process. The modification may include ionic or covalent binding of a compound, such as a bioactive agent or drug. Other modifications include controlled acetylation or hydrolysis reactions, in order to adjust the DA of the gel, thereby controlling mechanical properties, biodegradation, and biocompatibility. Most preferable is a hydrolysis (deacetylation) reaction leading to products having a low to moderate DA which further increases the mechanical strength. Hydrolysis may be performed by storage of the gel in concentrated alkaline solutions at elevated temperatures, such as for example in 40% aqueous sodium hydroxide solution at 110°C for 2 hours. More generally, hydrolysis may be performed by storage of N-acetylchitosan in a 10-50% aqueous alkaline solution at 50-120°C for up to 4 hours. Preferably, hydrolysis may be performed using a 30-50% aqueous alkaline solution at 60-110°C for 1-2 hours. Hydrolysis.may also be performed in several cycles in order to further decrease the degree of acetylation and improve the mechanical strength. Preferably, 1-2 cycles of hydrolysis may be used.

The medical device according to the present invention may contain additives, allowing the article to be designed to the specific requirements. Such additives may include acids, bases, plasticizers, fillers, dyes, porogens, contrast agents, microparticles, nanoparticles, bioactive agents and drugs. Such additives may be added to the reaction mixture prior to gel formation, and/or may be soaked into the gel by storage of the gel in a solution of the additive prior to the drying process. Such additives may also be soaked into the bulk or coated onto the surface of the product after drying.

The medical device according to the present invention may further be modified after the drying process, by a method described above for the hydrogels, including ionic or covalent binding of a compound, such as a bioactive agent or drug, and controlled acetylation or hydrolysis reactions, in order to adjust the DA, thereby controlling mechanical properties, biodegradation, and biocompatibility. Most preferable is a hydrolysis (deacetylation) reaction leading to products having a low to moderate degree of acetylation which further increases the mechanical strength. Hydrolysis may be performed by storage of the dried product in concentrated alkaline solutions at elevated temperatures, such as for example in 40% aqueous sodium hydroxide solution at 110°C for 2 hours. More generally, hydrolysis may be performed by storage of N-acetylchitosan in a 10-50% aqueous alkaline solution at 50-120°C for up to 4 hours. Preferably, hydrolysis may be performed using a 30-50% aqueous alkaline solution at 60-110°C for 1-2 hours. Hydrolysis may also be performed in several cycles in order to further decrease the DA. Preferably, 1-2 cycles of hydrolysis may be used.

The medical device according to the present invention may also be modified by coating with a layer of a polymer or other compound, which may be applied from solution by one of the techniques well-known in the art, such as dipping or spraying. Thus for example, a layer of a biodegradable polymer may be formed on the surface of the medical device in order to control its properties, including mechanical strength, biocompatibility, and biodegradation. Suitable biodegradable polymers include, for example, those from the group of synthetic polyesters, such as homopolymers and copolymers based on glycolide, L-lactide, D,L-lactide, p-dioxanone, £-caprolactone; natural polyesters, such as those from the group of the polyhydroxyalkanoates, such as homopolymers and copolymers based on 3-hydroxybutyrate, 4-hydroxybutyrate, 3-hydroxyvalerate, 3-hydroxyhexanoate, 3-hydroxyoctanoate; polyorthoesters; polycarbonates; polyanhydrides; polyurethanes; polyphosphazenes; polyphosphoesters; polysaccharides; polypeptides; as well as derivatives, copolymers, and blends based on the abovementioned and any other group of bioresorbable polymers. Other suitable polymers include those which may be dissolved under physiological conditions, such as homopolymers or copolymers based on vinyl alcohol, vinyl acetate, N-vinyl pyrrolidone, ethylene glycol, propylene glycol, polysaccharides, polypeptides, as well as derivatives, copolymers, and blends based on the aforementioned and any other group of biodissolvable polymers or combinations of biodegradable and biodissolvable polymers. Furthermore, it is possible to coat the device with a non-degradable or non-dissolvable polymer for specific applications, which require to prevent degradation or dissolution.

The polymer layer may further contain additives, including acids, bases, plasticizers, fillers, dyes, porogens, contrast agents, microparticles, nanoparticles, bioactive agents and drugs. Such additives may be added to the polymer solution prior to the coating process. In another example, a layer of a contrast agent may be formed on the surface of the device after its fabrication. For example, a layer of barium sulfate may be formed by dipping the device into an aqueous solution of a barium salt, followed by dipping into an aqueous solution containing sulfate ions, thereby forming a layer of barium sulfate on the surface of the device. In yet another example, a layer of a bioactive agent or drug may be formed on the surface of the device. For example, a layer of a bioactive agent or drug may be applied to the surface using an aqueous solution or organic solvent, followed by drying. In yet another example of modification of the medical device based on Nacetylchitosan, an additional layer of N-acetylchitosan gel may be applied to the surface of the device and may be dried for shape-fixation. These steps may be repeated several times to fabricate a multilayered device. The N-acetylchitosan layers may have different properties such as different DAs in order to define individual mechanical, biocompatibility, and biodegradation properties of individual layers. The N-acetylchitosan layers may be modified by techniques as described above or may contain additives as those described above. Such additives may also be embedded between the layers. In such a design, the additive will be applied to the surface of one layer of the device before adding the next layer of N-acetylchitosan gel. The subsequent drying process of this outer layer will lead to the incorporation of the additive between the layers.
The biodissolution process of a medical device that is made completely or in part of N-acetylchitosan may be controlled by
adjusting the pH of the physiological environment that is in contact with the medical device. N-Acetylchitosan becomes, in strong dependence on the DA, soluble under moderate acidic conditions, with a dissolution pattern that may be accompanied by swelling and/or incomplete disintegration, and that may be dependent on the presence of electrolytes and the availability of liquid as well as flow conditions at the application site. Therefore, medical devices based on N-acetylchitosan require well-defined DAs in order to establish their capability of being biodissolvable under controlled conditions.

For example, a medical device made of N-acetylchitosan that is temporarily used in urological applications, such as a urological stent, may be biodissolved by moderately decreasing the urine pH to slightly acidic. In a healthy human, the pH of the urine normally varies between 6.5 and 8. A temporary medical device in contact with urine should maintain its stability in this pH range for the time it is needed and, at the desired time, disintegrate within a short period of time, triggered by a pH change to values that allow for dissolution of the device. It is of importance that the pH to trigger the disintegration of the device can easily be adjusted by the physician or patient and is well tolerable. Preferably, a temporary device would disappear at a moderate pH of 4.5-6.0, more preferably at 5.0-5.5, to avoid premature disintegration due to naturally occuring variations of the pH and to limit unhealthy condition due to low pH.

As already outlined, the disintegration of a urological stent should not be accompanied by a significant swelling of the tube wall, causing tissue compression and irritation at the site of implantation, nor blockage of the tube lumen, leading to loss of functionality of the device. Additionally, any obstruction of an opening inside the body due to swelling of the degrading tube or due to fragments or particles that are cleaved off should be avoided. It would therefore be highly desirable to allow for a surface dissolution mechanism that would lead to a continuous decrease in the wall thickness thereby avoiding tube swelling and lumen obstruction, and it will not cause voluminous fragments to be formed in the course of disintegration.

Furthermore, the process of disintegration of a urological stent should generally occur within a relatively short period of time. This would allow the patient's urine to return to normal values and thereby reduce the risk of potential side-reactions due to an electrolytic imbalance. Additionally, a quick disintegration limits the risk of lumen blockage and obstruction due to accumulating pieces formed in the course of disintegration. Preferably, after adjusting the pH to the selected acidic value, disintegration of a urological device should be finished within less than two days, and more preferably, within less than 24 h, independently on the size of the device.

A urological stent made of N-acetylchitosan that fulfills all the aforementioned requirements under physiological conditions, including disintegration at pH 5.0-5.5, dissolution by surface-erosion, complete disappearance within less than 24 h, should have a DA of more than 8% and less than 21%, preferably of more than 10% and less than 18%, and particularly preferably of more than 12% and less than 16%.

In certain cases, it may be desired to allow for a step-wise disintegration of the urological device, by periodically adjusting the pH between acidic and neutral values. This feature of a device made of N-acetylchitosan is particularly interesting for drug-release applications, to allow for controlling the times and amounts of a drug to be released from the device.

Similar considerations as those above can be applied to tubular devices used in other applications than urology, such as gastrointestinal, biliary and pulmonary stents. Generally, the usage of an N-acetylchitosan device, including those of other shapes than tubes, may allow for controlled biodissolution in any case where the pH of the surrounding environment can be adjusted.

### EXAMPLES

### 1. Fabrication of N-acetylchitosan tube

An equal volume of ethanol and a 2fold molar amount of acetic anhydride were added to a 4% solution of chitosan (DA = 14%, viscosity of 1% solution in 1% acetic acid = 226 mPas) in 2% aqueous acetic acid, the chitosan solution containing an equal mass amount (to chitosan) of fine-dispersed barium sulfate powder (grain size appr. 1.0 µm). The reaction mixture was injected into a cylindrical mold (inner diameter 6.0 mm), which contained a fixed central cylindrical core (outer diameter 1.0 mm). After 24 h, during which syneresis occurred, the hydrogel tube formed was removed from the mold, washed with water, air-dried, and hydrolyzed, using 40% NaOH at 110°C for 4 h, resulting in an N-acetylchitosan tube of DA = 15%.

### 2. Dissolution of N-acetylchitosan tube in artificial urine

N-Acetylchitosan tubes fabricated as descibed in Example 1 were placed in a dynamic flow apparatus, comprising a peristaltic pump, a 500 ml reservoir containing 400 ml of artificial urine with a composition as described by McLean et al., "An in vitro ultrastructural study of infectious kidney stone genesis" Infect. Immun. 1985; 49; p. 805 (without usage of tryptic soy broth) that had its pH adjusted to 5.0, silicon connection tubing as well as Tygon tubing where the sample tubes were placed. The assembling, except of the peristaltic pump, was placed in an oven which was adjusted to 37°C. The artificial urine was pumped at a flow rate of 2 ml/min through the tubing containing the sample tubes, and pictures were taken every hour to follow the dissolution process (see Figure 1). Results from the dissolution testing are exemplified in Table 1.

**Table 1. Disintegration of N-acetylchitosan samples in artificial urine under dynamic flow conditions (2 ml/min, pH 5.0, 37°C).**

| Sample No. | Hydrolysis time (h) | DA (%, by NMR) | Dissolution time (h) | Disintegration pattern/ sample appearance |
|---|---|---|---|---|
| | | | | |
| 94 | 4 | 21.7 | insoluble | sample swollen |
| 95 | 4 | 19.2 | 8 | surface-erosion |
| 96 | 4 | 15.2 | 6 | surface-erosion |
| | | | | |
| 105 | 4 | 20.7 | 5 | surface-erosion |
| 106 | 4 | 13.0 | 5 | surface-erosion |
| 107 | 4 | 7.2 | incomplete | surface-erosion |
| 108 | 4 | 5.5 | insoluble | sample unchanged |
| | | | | |
| 115 | 4 | 17.6 | 5 | surface-erosion |
| 116 | 4 | 13.3 | 5 | surface-erosion |
| 117 | 4 | 6.8 | incomplete | surface-erosion |
| 118 | 4 | 5.2 | insoluble | sample unchanged |

## Claims

1. A stent, a catheter or a drug delivery device made at least partially of N-acetylchitosan having a degree of acetylation of more than 12% and less than 16%.

2. Use of N-acetylchitosan having a degree of acetylation of more than 12% and less than 16% in the manufacture of a stent, a catheter or a drug delivery device.

## Patentansprüche

1. Ein Stent, ein Katheter oder eine Vorrichtung zur Wirkstoffabgabe, zumindest teilweise aus N-Acetylchitosan bestehend, das einen Acetylierungsgrad von mehr als 12% und weniger als 16% aufweist.

2. Verwendung von N-Acetylchitosan, das einen Acetylierungsgrad von mehr als 12% und weniger als 16% aufweist, bei der Herstellung eines Stents, eines Katheters oder einer Vorrichtung zur Wirkstoffabgabe.

## Revendications

1. Stent, cathéter ou dispositif d'administration de médicaments fabriqué au moins partiellement en N-acétylchitosane possédant un degré d'acétylation supérieur à 12 % et inférieur à 16 %.

2. Utilisation de N-acétylchitosane possédant un degré d'acétylation supérieur à 12 % et inférieur à 16 % dans la fabrication d'un cathéter ou dispositif d'administration de médicaments.
